(19) **Europäisches Patentamt / European Patent Office / Office européen des brevets**

(11) **EP 1 483 974 B1**

(12) **EUROPEAN PATENT SPECIFICATION**

(45) Date of publication and mention
of the grant of the patent:
**17.08.2011 Bulletin 2011/33**

(51) Int Cl.:
***A23K 1/16*** (2006.01)     ***A23K 1/18*** (2006.01)

(21) Application number: **03744013.8**

(86) International application number:
**PCT/JP2003/002701**

(22) Date of filing: **07.03.2003**

(87) International publication number:
**WO 2003/075679 (18.09.2003 Gazette 2003/38)**

(54) **ADDITIVE FOR LIVESTOCK FEED**

ZUSATZSTOFF FÜR NUTZTIERFUTTER

ADDITIF DESTINE A DES ALIMENTS POUR LE BETAIL

(84) Designated Contracting States:
**AT BE BG CH CY CZ DE DK EE ES FI FR GB GR HU IE IT LI LU MC NL PT RO SE SI SK TR**

(30) Priority: **08.03.2002 JP 2002063881**

(43) Date of publication of application:
**08.12.2004 Bulletin 2004/50**

(73) Proprietor: **Yukiguni Maitake Co., Ltd.
Minami Uonuma-gun,
Niigata 949-6695 (JP)**

(72) Inventors:
• **OHDAIRA, Yasuo
Minamiuonuma-gun, Niigata 949-6631 (JP)**
• **IMAIZUMI, Nagao
Setagaya-ku, Tokyo 157-0062 (JP)**
• **TAKEYAMA, Masahide
Saitama-shi, Saitama 331-0063 (JP)**

(74) Representative: **Denison, Christopher Marcus et al
Mewburn Ellis LLP
33 Gutter Lane
London
EC2V 8AS (GB)**

(56) References cited:
EP-A- 1 155 623    JP-A- 3 076 539
JP-A- 2000 032 924    JP-A- 2000 125 778
JP-A- 2000 333 617    JP-A- 2001 069 921
JP-B- 3 260 329

• YUICHI SUZUKI ET AL.: 'Maitake saibai shiyo sumi baichi o tenka shita shiryo ni yoru nyuyoshu kyoseigyu no hiiku seiseki' BULLETIN OF BEEF CATTLE SCIENCE no. 60, 1995, pages 22 - 24, XP002975359

**Description**

TECHNICAL FIELD

The present invention relates to a feed additive for domestic animals, feed for domestic animals, and a method of raising domestic animals. More specifically, the present invention relates to a feed additive for domestic animals, feed for domestic animals, and a method of raising domestic animals, which reduce the number of deaths of domestic animals, increase the rate of raising and enable production of meat with high safety and good flavor.

BACKGROUND ART

In recent years, as competition in producing domestic animals increases, economical and efficient production has been required. Given a current situation, it is hard to say that comfortable environments for domestic animals to be raised in are always provided. As a result, animals suffer from stress, so that they tend to develop diseases such as infectious diseases.

Conventionally, drugs such as antibiotics are used as measures against diseases and particularly against infectious diseases. However, such drugs are unfavorable because they reside in the body, regular addition of antibiotics invites the emergence of resistant microbes, or the like. Currently, such drugs tend not to be used when possible.

For pet animals (pets), various pet foods have been developed, but the purposes thereof are to mainly prevent obesity resulting from overeating, diabetes, and the like.

However, feed for domestic animals, which aims at production of meat or the like, is completely different from so-called pet foods, so that the following points must be considered.

There is a need to satisfy conditions such as:

1) since domestic animals are often raised in a group, feed for domestic animals must be acceptable for the entire group of the animals;
2) feed for domestic animals must relieve stress in a group and prevent the spread of infectious diseases in a group, so as to improve the rate of raising;
3) particularly in a state of raising a group of domestic animals, once resistant microbes emerge, diseases easily spread, and thus feed for domestic animals must not allow the emergence of resistant microbes;
4) feed for domestic animals must make the flavor or the like of meat better rather than adversely affect the same, and be safe;
5) in view of economic efficiency and the like, feed for domestic animals must exert its action in as small a quantity as possible; and
6) a stable supply of feed for domestic animals must always be possible. Currently, feeds satisfying all of these conditions are extremely limited under the present situation.

SUMMARY OF THE INVENTION

An object of the present invention is to provide a feed additive for domestic animals, feed for domestic animals, and a method for raising domestic animals, which relieve the stress of domestic animals and prevent the death thereof so as to increase the rate of raising and enable production of meat with high safety and good flavor.

As a result of intensive studies to achieve the above object, we have discovered that the "Maitake" mushroom (Grifola) combined with a yeast-derived substance satisfies the above conditions, and thus have completed the present invention.

That is, the present invention relates to:

(1) a feed additive for domestic animals, comprising a Grifola-derived substance selected from one or more of dried Grifola, dried Grifola powder, and a Grifola extract combined with a yeast-derived substance, as defined in claim 1.
(2) the feed additive for domestic animals of (1), wherein the dried Grifola is produced by heating and drying fresh Grifola by gradually increasing the temperature stepwise initially from approximately 60°C to finally approximately 80°C;
(3) the feed additive for domestic animals of (1), wherein the Grifola extract is a Grifola extractive obtained by adding an alcohol to a water extract of fresh or dried Grifola or/and dried Grifola powder, leaving the resultant to stand, removing matter floating on or in the solution or adhering to the wall surface of a vessel, and concentrating the resultant;
(4) the feed additive for domestic animals of (1), wherein the yeast-derived substance is a dried yeast cell wall;
(5) the feed additive for domestic animals of (1), wherein and the Grifola-derived substance and the yeast-derived substance are mixed to result in a proportion of the former substance to the latter substance that is between 0.05 : 1.0 and 3.0 : 1.0;

(6) the feed additive for domestic animals of (5), wherein the yeast-derived substance is a dried yeast cell wall;

(7) the feed additive for domestic animals of any one of (1) to (6), wherein the domestic animals are chickens, swine, or cattle;

(8) a feed, comprising 0.05% by weight to 1% by weight of the feed additive for domestic animals of any one of (1) to (6) added thereto;

(9) a method for raising domestic animals, comprising providing 5 to 600 mg per day of the feed additive for domestic of any one of (1) to (6) per kg body weight of a domestic animal;

(10) the method for raising domestic animals of (9), wherein the domestic animals are chickens, swine, or cattle;

(11) a method for producing meat of domestic animals with good flavor, comprising providing the feed additive for domestic animals of any one of (1) to (6) to domestic animals;

(12) a method for producing meat of domestic animals with good flavor, comprising providing 5 to 600 mg per day of the feed additive for domestic animals of any one of (1) to (6) per kg body weight of a domestic animal; and

(13) the method for producing the meat of domestic animals of (11) or (12), wherein the domestic animals are chickens, swine, or cattle.

In addition, mixing of Grifola or a Grifola extract into pet foods for the purpose of improving and maintaining the glow of a pet hair coat or preventing lifestyle-related diseases such as obesity and diabetes is disclosed in JP Patent Publication (Kokai) No. 8-38069 A (1996) and JP Patent Publication (Kokai) No. 2001-69921 A (2001).

However, pets such as dogs and cats are placed in circumstances that can be said to be the intake of excess nutrition. Thus these inventions are significantly different from the present invention in terms of types of animals and purposes thereof, because the present invention aims at increasing the rate of raising chickens, swine, cattle, and other domestic animals for meat production. Furthermore, the feed additive and the feed of the present invention have a purpose differing from those of the above known examples in that they can have an effect not only on the improvement of animals' own health but also on the qualities of animal meat, including safety, flavor, and the like.

The use of Grifola in animal feed for the purpose of weight gain is disclosed in EP 1 155 623 and in Y. Suzuki et al.: 'Maitake saibai shiyo sumi baichi o tenka shita shiryo ni yoru nyuyoshu kyoseigyu no hiiku seiseki' Bulletin of Beef cattle science no. 60, 1995, page 22-24. The use of a yeast culture in a feed blend for pregnant cow is disclosed in JP3076539. The use of a bioactive agent comprising enzymes having adsorption activity for a mineral supplying agent, as active agent for the purpose of preventing disease and promoting growth of fish, shell fish, livestock and pets is disclosed in JP2000333617.

In the present invention, all types of Grifola including "Maitake" (Grifola frondosa), "Shiromaitake" (Grifola albicans), "Choreimaitake" (Dendropolyporus umbellatus), "Tonbimaitake" (Grifola gigantea), and the like can be used. In addition, both mycelia and fruit bodies of these types of Grifola can be used. Recently, artificial cultivation of the fruit bodies of "Maitake" (Grifola frondosa) has been made possible. It is preferable to use the fruit bodies of Grifola in terms of ensuring a stable supply of raw materials.

As dried Grifola, any Grifola prepared by sun drying, hot air drying, or freeze drying can be used. As a result of studies, it has been shown that dried products prepared by, for example, drying fresh Grifola by gradually increasing the temperature stepwise initially from approximately 60°C to finally approximately 80°C are preferable because of their good active oxygen-scavenging activity. Thus, the applicant has obtained a patent (Japanese Patent No. 3260329).

As a Grifola-derived substance obtained as described above, dried Grifola, that is, Grifola in a dried form, can be used as it is, or can also be used in the form of small chips, small pieces, or fine pieces after appropriate grinding. However, it is more general to grind dried Grifola into powder using a milling apparatus or the like for use, because this leads to a broader application range. In addition, depending on the state of feed to which Grifola is added, Grifola powder can be appropriately selected from those with large particle sizes to Grifola finer powder with small particle sizes.

As a Grifola extract, extracts produced by standard methods using water, an alcohol, or the like can be used. The applicant has elucidated that a Grifola extractive obtained by adding an alcohol to the water extract of fresh or dried Grifola or dried Grifola powder, leaving the resultant to stand, removing matter floating on or in the solution or adhering to the wall surface of a vessel, and concentrating the resultant is preferable because of its good immunopotentiating action and active oxygen-scavenging activity (Japanese Patent Nos. 2859843 and 3260329).

The extraction method is performed at a temperature between room temperature and 135°C for 15 minutes to 3 hours. The short-time extraction is performed at 100°C or higher under pressure; for example, at about 120°C for about 30 minutes to about 1 hour under an atmospheric pressure between 1 and 2 using an autoclave. As water, any type of water such as distilled water, purified water, ion exchanged water, tap water, or natural water can be used. The amount of water used for the extraction is, for example, from about 4 volumes to about 20 volumes, based on 1 weight of dried Grifola or dried Grifola powder. When fresh Grifola is used, water is used in an amount from about 2 volumes to about 10 volumes, based on 1 weight of the same.

As an alcohol, methanol, ethanol, and the like can be used. An alcohol may be added to the extract in a final concentration between 20% and 70% by volume. An alcohol with water content between 0% and 50% can be used. After the addition of the alcohol, the resultant solution is left to stand for 1 hour to 20 hours at a temperature between 1°C and 25°C, whereby matter floating on or in the solution or adhering to the wall surface of a vessel appears. The matter is then collected and removed from the solution by filtration, pipetting, scooping up with a meshed material, or similar methods.

The thus obtained extract can be used as dried extractive powder by conducting any conventional drying means such as concentration drying, spray drying, vacuum drying, or freeze drying. Furthermore, the extract can also be used after purification.

The dried Grifola, dried Grifola powder, and Grifola extract obtained as described above can be added independently to feed. To enable thorough mixing of the Grifola added to the feed, Grifola powder, Grifola extract or Grifola in the dosage form of powders mixed with an expander and a lubricant, granules, pellets, or the like prepared by standard methods can also be added to the feed.

When a case of using Grifola in the form of powder is explained as an example, an expander and a lubricant are added to the dried Grifola powder or the Grifola extract, and then the resultant is thoroughly mixed. At this time, if necessary, substances other than a Grifola-derived substance can be added.

As an expander such as lactose, starch, dextrin, or the like can be used. As a lubricant, light liquid paraffin or the like can be used. Particularly when the Grifola extract is used, it is preferable to mix the Grifola extract with an expander, because the Grifola extract becomes easily moistened.

A Grifola-derived substance contains β-glucan and has immunopotentiating action. As a result of searching for a substance having similar immunopotentiating action, we have discovered that it is more preferable to mix in a yeast-derived substance.

As a yeast-derived substance, those fermented by yeast (<u>Saccharomyces cerevisiae</u>) used in bread production, sake brewing, wine brewing or top fermentation beer brewing using a sugar-containing resource, so-called beer yeast obtained upon bottom fermentation beer brewing using yeast, and the like can be used.

Furthermore, through the use of various types of yeast as raw materials, yeast-derived substances such as yeast extractives extracted by autolysis or the addition of enzymes, and furthermore, dried yeast cell walls (YCW marketed by Tanabe Seiyaku Co., Ltd.) obtained by separating yeast cell walls rich in polysaccharides such as glucan and mannan can be used.

Among yeast-derived substances, it is preferable to use dried yeast cell walls, particularly in terms of their good in vivo immunopotentiating action.

When a feed additive is produced by mixing a Grifola-derived substance with a yeast-derived substance, any compounding ratios can be selected. As a standard, it is better to select a compounding ratio of a Grifola-derived substance to a yeast-derived substance that is between 0.05 : 1.00 and 3.00 : 1.00. For example, when dried Grifola extract powder is used as a Grifola-derived substance, mixing can be performed based on a mixing ratio of the dried Grifola extract powder to a yeast-derived substance that is between about 0.05 : 1.00 and about 0.50 : 1.00. Alternatively, when dried Grifola or dried Grifola powder is used, mixing can be performed based on a mixing ratio of the dried Grifola or the dried Grifola powder to a yeast-derived substance broadly ranging from about 0.20 : 1.00 to about 3.00 : 1.00.

Feed additives obtained as described above are used by appropriately adding the additives to animal feed. The addition percentage is 0.05% by weight or more. The upper limit is the amount of the additive that an animal can ingest. In the case of domestic animals, economic efficiency should also be considered. Thus, it is considered reasonable that about 1% by weight be regarded as an upper limit.

For example, when 0.05% by weight to 1% by weight of the feed additive of the present invention is mixed into feed, standards regarding the amount of the additive to be provided per day to chickens, swine, cattle, or the like are as shown below.

Table 1

| Standards regarding the amount of the additive to be provided | | | | |
|---|---|---|---|---|
| | Body weight | Feed (amount/day) | 0.05% to 1% by weight of feed additive mixed in the feed (per animal) | 0.05% to 1% by weight of feed additive mixed in the feed (per kg) |
| Chicken (broiler) | 3 kg | 175 g | 87.5 to 1750 mg | 29.2 to 583.3 mg |
| Swine | 100 kg | 3.5 kg | 1.75 to 35 g | 17.5 to 350.0 mg |
| Cattle (for fattening) | 600 kg | 7.5 kg | 3.75 to 75 g | 6.3 mg to 125.0 mg |

As understood from Table 1, in the case of adding 0.05% by weight to 1% by weight of the feed additive to feed, the feed additive is provided in an amount within a range between approximately 5 mg and 600 mg per kg body weight, regardless of the type of domestic animal. This can be said to be a safe and appropriate amount.

"Domestic animals" used in the present invention include animals for use in production of meat in addition to chickens, swine, cattle, domestic ducks, and sheep. In particular, the present invention is effective for chickens, swine, and cattle.

BEST MODE FOR CARRYING OUT THE INVENTION

Next, examples will be given to specifically explain the present invention.

[Example 1] Production of Grifola-derived substance

(1) Dried Grifola and dried Grifola powder

Artificially-cultivated fruit bodies of fresh Grifola frondosa were lined on the racks of a drying room with racks, and then dried by blowing hot air at approximately 60°C to approximately 80°C. By raising the temperature stepwise initially from 60°C to finally 80°C for almost 1 day so as to perform heating and drying, dried fruit bodies of Grifola frondosa were obtained. Subsequently, the dried Grifola frondosa was powdered with a milling apparatus.

(2) Grifola extract

10 kg of the dried fruit bodies of Grifola frondosa powder (hereinafter referred to as dried Grifola powder) was treated under pressure at 120°C with 100 L of purified water for 30 minutes, and then filtered, thereby obtaining 60 L of a blackish-brown extract. The solution was concentrated under reduced pressure to 25 L. When 28 L of 95% ethanol was added to the solution at room temperature, and then the resultant was left to stand for approximately 18 hours, brownish-red matter floating on or in the solution or adhering to the wall surface of the vessel was generated.

This matter was scooped up and removed with a metal mesh, thereby obtaining a brown solution. Alcohol was removed from the solution under reduced pressure, and then the resultant was concentrated under reduced pressure (24 to 42 kPa) at 70°C to 80°C until the Brix value of the solution became approximately 50%, thereby obtaining a heavy blackish-brown solution.

The solution was spray-dried using a rotating-disk-type spray dry system (centrifugal spray drying), thereby obtaining 1.6 kg of brown Grifola extractive powder peculiar to Grifola.

[Example 2] Preparation of feed additive

|  |  |  |
|---|---|---|
| Feed additive 1 (not according to the invention) | | |
|  | Dried Grifola powder | 5 kg |
|  | Lactose | 5 kg |
|  | Total | 10 kg |
| Feed additive 2 | | |
|  | Dried Grifola powder | 3 kg |
|  | Dried yeast cell wall (YCW) | 4 kg |
|  | Lactic acid bacteria | 0.05 kg |
|  | Light liquid paraffin | 0.2 kg |
|  | Lactose | remainder |
|  | Total | 10 kg |
| Feed additive 3 | | |
|  | Grifola extractive powder | 0.2 kg |
|  | Dried yeast cell wall (YCW) | 4.0 kg |
|  | Light liquid paraffin | 0.2 kg |

(continued)

| Feed additive 3 | |
| --- | --- |
| Lactose | remainder |
| Total | 10 kg |

Items in the above prescription were uniformly mixed well, so that each feed additive was prepared.

The additives prepared as described above can be directly mixed into feed and then used. If necessary, the additives can be prepared in the form of granules, soft pellets, dry pellets, or the like by standard methods, and then used.

[Example 3] Raising test results and flavor and organoleptic test for produced meat

(1) Raising test results for chickens

0.2% by weight of the feed additive 1 was added to the feed for test group 1, and 0.2% by weight of the feed additive 2 was added to the feed for test group 2. A group provided with feed to which no feed additive had been added was used as a control group. The test was conducted from September to October. As a result, results as shown in the following Table 2 were obtained.

Table 2

| Raising test results for chickens | | | |
| --- | --- | --- | --- |
| | Test Group 1 | Test Group 2 | Control Group |
| Number of incoming chicks | 12750 | 12750 | 12450 |
| Number of chickens shipped | 12107 | 12359 | 11227 |
| Rate of raising | 94.96 | 96.93 | 90.18 |
| Average body weight (kg) | 2.919 | 2.934 | 2.911 |
| Age in day | 53.35 | 52.69 | 53.06 |
| Feed-requiring rate | 2.085 | 2.054 | 2.206 |
| PS* | 249.2 | 262.8 | 224.3 |
| Waste ratio | 1.51 | 1.44 | 1.80 |

$$*PS = \frac{\text{Rate of raising} \times \text{Average body weight}}{\text{Age in day} \times \text{Feed-requiring rate}} \times 100$$

As is also clear from the above results, test groups 1 and 2 showed excellent results in terms of rate of raising, PS value, waste ratio, and the like compared with those of the control group. This was the result of a low number of deaths due to stresses such as infectious diseases.

(2) Flavor test results

Five chickens were randomly sampled from the chickens shipped of test group 2 and the control group, respectively, and then sacrificed. For odor and taste (the smell peculiar to chicken meat) of the breast meat, a total of 20 subjects consisting of 14 male subjects and 6 female subjects, whose age ranged from 30 to 70 years old, were subjected to a simple 3-stage organoleptic test involving comparison of the test group with the control group. Thus, the following results were obtained.

I. Raw meat Odor (Comparison of the smell peculiar to chicken)

     (1) Smelled unsavory compared with the control: 1 out of 20 subjects
     (2) Unable to say which was which: 7 out of 20 subjects
     (3) Improved compared with the control: 12 out of 20 subjects

II. Directly boiled meat without seasoning

Odor (Comparison of the smell peculiar to chicken)

     (1) Smelled unsavory compared with the control: 1 out of 20 subjects
     (2) Unable to say which was which: 5 out of 20 subjects
     (3) Improved compared with the control: 14 out of 20 subjects

Taste (Comparison of the taste peculiar to chicken)

     (1) Tasted unsavory compared with the control: 1 out of 20 subjects
     (2) Unable to say which was which: 4 out of 20 subjects
     (3) Improved compared with the control: 15 out of 20 subjects

III. Directly grilled meat without seasoning

Odor (Comparison of the smell peculiar to chicken)

     (1) Smelled unsavory compared with the control: 0 out of 20 subjects
     (2) Unable to say which was which: 2 out of 20 subjects
     (3) Improved compared with the control: 18 out of 20 subjects

Taste (Comparison of the taste peculiar to chicken)

     (1) Tasted unsavory compared with the control: 0 out of 20 subjects
     (2) Unable to say which was which: 3 out of 20 subjects
     (3) Improved and tasted good compared with the control: 17 out of 20 subjects

As shown above, it was revealed that the test group was improved in terms of odor and taste compared with those of the control group.

(3) Results of the effect of body weight gain in weaner piglets

Weaner piglets were observed, concerning the presence or the absence of an effect of body weight gain, wherein a group to which feed comprising 0.2% by weight of feed additive 2 mixed therein had been provided and a control group to which only feed had been provided were compared.

(Test groups)

| Test group: 55 female piglets | Feed comprising 0.2% by weight of feed additive 2 mixed therein |
| Control group: 27 female piglets | Only feed |
| (Test period) | |
| 27- (at the time of weaning) to 90-day-old piglets | |
| (Feed during the test period) | |
| I. 27 days to 60 days after birth | Synthetic milk A |
| II. 61 days to 70 days after birth | Synthetic milk B |
| III. 71 days to 90 days after birth | Feed for piglets |

Table

| Changes in average body weight | | |
| --- | --- | --- |
| | At start time of test | At end time of test |
| Test group | 7.92 kg | 44.2 $\pm$ 5.1 kg |
| Control group | 7.92 kg | 41.4 $\pm$ 6.8 kg |

From the above results, clear tendencies of body weight gain were observed in the test group.

Industrial Applicability

The feed additive for domestic animals, the feed for domestic animals, or the method for raising domestic animals of the present invention is safe for domestic animals, enhances the immune strength of domestic animals, reduces the number of deaths of domestic animals due to stress, infectious diseases, or the like so as to increase the rate of raising, and has an effect of making the flavor of the meat of domestic animals better.

**Claims**

1. A feed additive for increasing the rate of raising of domestic animals, comprising a Grifola-derived substance selected from among one or more of dried Grifola, dried Grifola powder, and a Grifola extract and a yeast-derived substance mixed therein, wherein the animals are animals for use in production of meat.

2. The feed additive of claim 1, wherein the yeast-derived substance is a dried yeast cell wall.

3. The feed additive of claim 1, wherein the Grifola-derived substance and the yeast-derived substance are mixed to result in a proportion of the former substance to the latter substance that is between 0.05 : 1.0 and 3.0 : 1.0.

4. The feed additive of claim 3, wherein the yeast-derived substance is a dried yeast cell wall.

5. The feed additive of any one of claims 1 to 4, wherein the domestic animals are chickens, swine, or cattle.

6. A feed for domestic animals, wherein the animals are animals for use in production of meat, the feed comprising 0.05% by weight to 1% by weight of the feed additive of any one of claims 1 to 4.

7. A method for raising domestic animals, comprising providing 5 to 600 mg per day of the feed additive of any one of claims 1 to 4 per kg body weight of a domestic animal, wherein the animals are animals for use in production of meat.

8. The method of claim 7, wherein the domestic animals are chickens, swine, or cattle.

9. A method for producing meat of domestic animals with good flavor, comprising providing the feed additive of any one of claims 1 to 4 to domestic animals, wherein the animals are animals for use in production of meat..

10. A method according to claim 9, comprising providing 5 to 600 mg per day of the feed additive of any one of claims 1 to 4 per kg body weight of a domestic animal.

**11.** The method of claim 9 or 10, wherein the domestic animals are chickens, swine, or cattle.

**Patentansprüche**

**1.** Futtermittelzusatz zur Steigerung der Aufzuchtrate von Nutztieren, umfassend eine von Grifola abgeleitete Substanz, die aus einem oder mehreren von getrockneter Grifola, getrocknetem Grifola-Pulver und Grifola-Extrakt ausgewählt ist, und eine darin eingemischte, von Hefe abgeleitete Substanz, wobei die Tiere Tiere zur Nutzung bei der Fleischproduktion sind.

**2.** Futtermittelzusatz nach Anspruch 1, worin die von Hefe abgeleitete Substanz getrocknete Hefezellwand ist.

**3.** Futtermittelzusatz nach Anspruch 1, worin die von Grifola abgeleitete Substanz und die von Hefe abgeleitete Substanz so vermischt sind, dass ein Verhältnis zwischen ersterer Substanz und letzterer Substanz von zwischen 0,05:1,0 und 3,0:1,0 vorliegt.

**4.** Futtermittelzusatz nach Anspruch 3, worin die von Hefe abgeleitete Substanz getrocknete Hefezellwand ist.

**5.** Futtermittelzusatz nach einem der Ansprüche 1 bis 4, worin die Nutztiere Hühner, Schweine oder Rinder sind.

**6.** Futtermittel für Nutztiere, worin die Tiere Tiere zur Nutzung bei der Fleischproduktion sind, wobei das Futtermittel 0,05 Gew.-% bis 1 Gew.-% eines Futtermittelzusatzes nach einem der Ansprüche 1 bis 4 umfasst.

**7.** Verfahren zur Aufzucht von Nutztieren, umfassend das Bereitstellen von 5 bis 600 mg eines Futtermittelzusatzes nach einem der Ansprüche 1 bis 4 pro Tag und kg Körpergewicht eines Nutztiers, wobei die Tiere Tiere zur Nutzung bei der Fleischproduktion sind.

**8.** Verfahren nach Anspruch 7, worin die Nutztiere Hühner, Schweine oder Rinder sind.

**9.** Verfahren zur Produktion von wohlschmeckendem Fleisch von Nutztieren, umfassend das Bereitstellen eines Futtermittelzusatzes nach einem der Ansprüche 1 bis 4 für Nutztiere, worin die Tiere Tiere zur Nutzung bei der Fleischproduktion sind.

**10.** Verfahren nach Anspruch 9, umfassend das Bereitstellen von 5 bis 600 mg eines Futtermittelzusatzes nach einem der Ansprüche 1 bis 4 pro Tag und kg Körpergewicht eines Nutztiers.

**11.** Verfahren nach Anspruch 9 oder 10, worin die Nutztiere Hühner, Schweine oder Rinder sind.

**Revendications**

**1.** Additif alimentaire pour augmenter le taux d'élévation d'animaux domestiques, comprenant une substance dérivée de Grifola sélectionnée parmi un ou plusieurs de Grifola séchée, poudre de Grifola séchée et un extrait de Grifola et une substance dérivée d'une levure mélangée avec celle-ci, où les animaux sont des animaux utilisés pour la production de la viande.

**2.** Additif alimentaire selon la revendication 1, où la substance dérivée d'une levure est une paroi cellulaire de levure séchée.

**3.** Additif alimentaire selon la revendication 1, où la substance dérivée de Grifola et la substance dérivée de levure sont mélangées pour obtenir une proportion de la première substance à la dernière substance qui est entre 0,05:1,0 et 3,0:1,0.

**4.** Additif alimentaire selon la revendication 3, où la substance dérivée de la levure est une paroi cellulaire de levure séchée.

**5.** Additif alimentaire selon l'une quelconque des revendications 1 à 4, où les animaux domestiques sont des poulets, porcs ou du bétail.

**6.** Aliment pour des animaux domestiques, où les animaux sont des animaux utilisés pour la production de viande, l'aliment comprenant 0,05% en poids à 1% en poids de l'additif alimentaire selon l'une quelconque des revendications 1 à 4.

**7.** Procédé pour élever des animaux domestiques, comprenant la fourniture de 5 à 600 mg par jour de l'additif alimentaire selon l'une quelconque des revendications 1 à 4 par kg de poids corporel d'un animal domestique, où les animaux sont des animaux utilisés pour la production de viande.

**8.** Procédé selon la revendication 7, dans lequel les animaux domestiques sont des poulets, porcs ou du bétail.

**9.** Procédé de production de viande à partir d'animaux domestiques d'une bonne saveur, comprenant la fourniture de l'additif alimentaire selon l'une quelconque des revendications 1 à 4 à des animaux domestiques, où les animaux sont des animaux utilisés pour la production de viande.

**10.** Procédé selon la revendication 9, comprenant la fourniture de 5 à 600 mg par jour de l'additif alimentaire selon l'une quelconque des revendications 1 à 4 par kg de poids corporel d'un animal domestique.

**11.** Procédé selon la revendication 9 ou 10, dans lequel les animaux domestiques sont des poulets, porcs ou du bétail.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8038069 A
- JP 2001069921 A
- EP 1155623 A
- JP 3076539 B
- JP 2000333617 B
- JP 3260329 B
- JP 2859843 B

**Non-patent literature cited in the description**

- **Y. Suzuki et al.** Maitake saibai shiyo sumi baichi o tenka shita shiryo ni yoru nyuyoshu kyoseigyu no hiiku seiseki. *Bulletin of Beef cattle science,* 1995, vol. 60, 22-24